# EUROPEAN PATENT SPECIFICATION

(11) **EP 0 806 925 B1**
(45) Date of publication and mention of the grant of the patent: **11.08.2004**
(21) Application number: 96903622.7
(22) Date of filing: 24.01.1996
(51) Int. Cl.: A61F 13/15, A61F 13/20, B32B 31/08

(54) **ABSORBENT ARTICLE**
ABSORBIERENDER ARTIKEL
ARTICLE ABSORBANT

(30) Priority: 02.02.1995 JP 73592195
(43) Date of publication of application: 19.11.1997
(73) Proprietor: THE PROCTER & GAMBLE COMPANY, Cincinnati, Ohio 45202 (US)
(72) Inventor: OSHIMA, Kenji, Shin-Osaka, Yodogawa-ku, Osaka 532 (JP); SHIKATA, Hiroaki, Higashinada-ku, Kobe 658 (JP)
(74) Representative: Veronese, Pancrazio
(86) International application number: PCT/US1996/000864
(87) International publication number: WO 1996/023471

(56) References cited:
- US-A- 4 701 177
- US-A- 5 308 346
- US-A- 5 346 486
- US-A- 5 387 210
- US-A- 5 413 569
- US-A- 5 490 847

## Description

### FIELD OF THE INVENTION

This invention relates to disposable absorbent articles, and more particularly, to a disposable absorbent article having a liquid pervious topsheet, a liquid impervious backsheet, and an absorbent core disposed intermediate the topsheet and the backsheet.

The term "absorbent articles" as used herein refers to sanitary napkins, pantiliners, and incontinence pads designed to absorb and retain body fluids, such as menses and urine, discharged from the human body when they are worn so as to cover the urogenital region.

### BACKGROUND OF THE INVENTION

As is well known, an absorbent article advantageously used as a sanitary napkin has a liquid pervious topsheet, a liquid impervious backsheet, and an absorbent core disposed intermediate the topsheet and the backsheet. Such an absorbent article should conform to the wearer's urogenital region, and be able to prevent leakage of body fluids, particularly in the lateral direction. From these points of view, the absorbent article is desired to be such that the topsheet, the core, and the backsheet extend upwardly inclinedly toward the longitudinal ends of the absorbent article at each of the longitudinally opposite end portions thereof, thereby assuming the shape of a cup as a whole, and that so-called cuffs extending upwardly inclinedly in a widthwise inwardly or outwardly are formed on both sides of the longitudinally central portion of the absorbent article.

The term "longitudinal" as used herein refers to a direction extending on the front and back of the body along the absorbent article worn in a required state covering the wearer's urogenital region (a direction extending substantially horizontally in the back-and-forth direction of the body when the absorbent article is laid, in a flat form, substantially horizontally below the crotch region of the body standing upright). The term "widthwise" as used herein refers to a direction extending on the right and left of the body along the absorbent article worn in a required state covering the wearer's urogenital region (a direction extending substantially horizontally in the right-and-left direction of the body when the absorbent article is laid, in a flat form, substantially horizontally below the crotch region of the body standing upright).

A first proposal in the prior art for making the absorbent article cup-shaped and forming cuffs, as stated above, is to shape the topsheet, the core and the backsheet themselves such that the absorbent article takes the cup-like form, and the topsheet and/or the backsheet forms the cuffs on both sides of the longitudinally central portion of the absorbent article, rather than shaping the topsheet, the core and the backsheet to an originally flat form. As the prior references disclosing this first proposal can be cited the specification and drawings of U.S. Patent 3,575,174, the specification and drawings of U.S Patent 4,678,527 (Japanese Laid-Open Patent Publication No 76156/86), and the specification and drawings of U.S. Patent 4,834,739 (Japanese Laid-Open Patent Publication No. 4371/90)

The absorbent article constructed in accordance with the first proposal in the prior art involves the following problems: (1) The cost of manufacturing considerably increases, because the topsheet, the core and the backsheet have to be shaped to a required form. (2) The free end of the cuff is contacted with the wearer's skin. Since the portion constituting the cuff has no stretching properties, it is highly likely to cause discomfort to the wearer, leading to a poor feeling when worn.

A second proposal in the prior art for making the absorbent article cup-shaped, as stated above, and forming the cuffs is to join elastically expansile and contractile band-like elastic pieces, in an elastically elongated state, to the topsheet and/or the backsheet on both sides in the widthwise direction of the absorbent article. As the band-like elastic piece, the use of a rubber strip or a thermoplastic elastomer strip has been proposed. In the absorbent article equipped with such band-like elastic pieces, the topsheet, core and backsheet are shaped like the cup, and the topsheet and/or the backsheet forms the cuffs, owing to the contracting action of the band-like elastic pieces As the prior references disclosing this second proposal can be cited the specification and drawings of U.S. Patent 4,579,556, the specification and drawings of U.S. Patent 4,701,177, the specification and drawings of U S Patent 4,758,241, the specification and drawings of U.S. Patent 4,770,657, the specification and drawings of U.S Patent 4,944,735 (Japanese Laid-Open Patent Publication No. 193461/85), the specification and drawings of U.S. Patent 5,032,121, the specification and drawings of U.S. Patent 5,074,856, the specification and drawings of U.S. Patent 5,234,422, the specification and drawings of U.S. Patent 5,308,346, the specification and drawings of U.S. Patent 5,312,386 (Japanese Laid-Open Patent Publication No. 277453/90), the specification and drawings of European Laid-Open Patent Application 557,047 (Japanese Laid-Open Patent Publication No. 220191/93). the specification and drawings of European Laid-Open Patent Application 534,488, the specification and drawings of European Laid-Open Patent Application 590,675, the specification and drawings of U.K Patent 2,195,541 (Japanese Laid-Open Patent Publication No. 122451/88), and the specification and drawings of International Laid-Open Patent Application 92/07536 (Japanese Laid-Open Patent Publication No. 501409/94).

In the absorbent article constructed in accordance with the second proposal in the prior art, elastic stretching properties are imparted to the absorbent article, because of the presence of the band-like elastic pieces formed of a rubber strip or a thermoplastic elastomer. This results in a better feeling when worn than that of the absorbent article constructed in accordance with the first proposal. However, the absorbent article following the second proposal poses the following problems: (1) The cost of manufacturing considerably increases, because the use of the band-like elastic piece made of a relatively expensive rubber strip or thermoplastic elastomer strip is required. (2) It is necessary to create in the topsheet a space for accommodating the band-like elastic pieces, and/or additionally dispose a means for joining the elastic pieces to the topsheet and/or the backsheet, as required. This adds to the cost of manufacturing.

A third proposal in the prior art for making the absorbent article cup-shaped, as stated above, and forming the cuffs is to thermally bond the longitudinally opposite ends and the widthwise outward portion of a thermoplastic elastic piece, in an elastically elongated state, to required sites on the upper surface of the topsheet joined, as required, to the core and the backsheet on each of both sides of the absorbent article. In the absorbent article equipped with such elastic pieces, the topsheet, core and backsheet are shaped like the cup owing to the contracting action of the elastic pieces. On both sides of the longitudinal central portion, the elastic pieces are caused to extend upwardly inclinedly in a widthwise inward direction, forming the cuffs. Such a third proposal is disclosed in Japanese Laid-Open Utility Model Publication No. 86323/93.

In the absorbent article constructed in accordance with the third proposal in the prior art, the thermoplastic elastic pieces themselves which are thermally bonded to the topsheet form cuffs on both sides of the longitudinally central portion. Moreover, thermal bonding can be carried out relatively simply. Thus, some improvement is achieved on the aforementioned problems facing the absorbent articles constructed in accordance with the first and second proposals. The absorbent article following the third proposal, however, presents with the following problems: (1) The elastic piece is in the form of an ordinary film, with its front and back being planar. Thus, it has a relatively large area of contact with the wearer's skin, and the feeling of the absorbent article when worn is still not satisfactory. (2) The thermoplastic elastic piece needs to be formed of a material having sufficient elasticity, and is thus relatively expensive, adding considerably to the cost of manufacturing (3) The thermoplastic elastic piece needs to be formed of a material having sufficient elasticity, which usually is considerably different from the material of the topsheet in terms of the molecular structure. Hence, it is difficult to thermally bond the thermoplastic elastic piece to the topsheet fully satisfactorily. (4) The core and the backsheet are joined to the topsheet to form the body of the absorbent article. In this state, the thicknesses at the longitudinally opposite end portions and the widthwise opposite end portions of the topsheet have been sharply vaned, because there is no core there Afterwards, the thermoplastic elastic pieces are thermally bonded to the topsheet, thus posing difficulty with the thermal bonding of the thermoplastic elastic pieces.

The present invention has been accomplished in the light of the above-mentioned facts. The first objective for the invention is to provide an absorbent article which takes the form of a cup as a whole, forms upwardly extending cuffs on both sides of the longitudinally central portion thereof, and yet produces a fully satisfactory feeling when worn.

The second objective for the present invention is to provide an absorbent article which takes the form of a cup as a whole, forms upwardly extending cuffs on both sides of the longitudinally central portion thereof, and yet produces a fully satisfactory feeling when worn, and additionally can be manufactured for a sufficiently low cost.

### SUMMARY OF THE INVENTION

The present invention provides an absorbent article in accordance with the appended claims.

According to the present invention for overcoming the first technical challenge, elastic pieces to be joined to at least one of the topsheet and the backsheet are formed from a web deformed at least partially in a non-planar configuration by machining.

That is, according to one aspect of the present invention, there is provided an absorbent article comprising a liquid pervious topsheet, a liquid impervious backsheet, an absorbent core disposed intermediate the topsheet and the backsheet, and a pair of band-like elastic pieces arranged on both sides of the absorbent article and joined to at least one of the topsheet and the backsheet in a longitudinally elastically elongated state; the topsheet, the backsheet, and the core being caused to extend upwardly inclinedly toward the longitudinal ends of the absorbent article at each of the longitudinally opposite end portions thereof, and the elastic pieces being caused to extend upwardly on both sides of the longitudinally central portion of the absorbent article, owing to the contracting action of the elastic pieces; wherein
each of the elastic pieces is formed from a web deformed at least partially in a non-planar configuration by machining.

In order to solve the second technical challenge as well as the first technical challenge, a thermoplastic polyolefin film lacking required elasticity prior to machining, preferably a polyethylene or polyethylene blended film, is used as the web formed into the elastic piece by machining. The machined synthetic resin film includes deformed portions deformed in a non-planar configuration by machining, and undeformed portions retained in a planar configuration. Furthermore, the undeformed portions are caused to extend longitudinally, and the contracting action of each of the elastic pieces is generated mainly by the undeformed portions. When the elastic pieces are subjected to an applied elongation, the undeformed portions undergo a substantially molecular-level deformation and the deformed portions undergo a substantially geometric deformation.

The area rate of the undeformed portions of each of the elastic pieces, calculated on the assumption that the elastic pieces are in a non-elongated state and the deformed portions are in a planar configuration, is 1 to 30%, preferably 3 to 20%, more preferably 5 to 12%. If the area rate of the undeformed portions is too large, discomfort the wearer feels upon contact with the skin increases. If the area rate of the undeformed portions is too small, by contrast, the contracting action for shaping the absorbent article like a cup tends to be diminished markedly.

Preferably, each of the elastic pieces is folded back along a longitudinally extending fold-back line to have a two-layer configuration, and the fold-back lines define the upper edges on both sides of the longitudinal central portion of the absorbent article. If the deformed portions and the undeformed portions of each of the elastic pieces extend longitudinally uninterruptedly in a widthwise side-by-side relationship, the fold-back line can be situated at the undeformed portion or the deformed portion. When the fold-back line is situated at the undeformed portion, the upper edge of the elastic piece (the fold-back line) becomes relatively sharp, providing a good contact with the wearers skin. When the fold-back line is situated at the deformed portion, the upper edge of the elastic piece (the fold-back line) becomes dull, giving the wearer a gentle feeling when wearing the absorbent article.

Preferably, at least part of each of the elastic pieces is disposed above the topsheet, the core is smaller than the topsheet, the longitudinally opposite ends of the core are located inwardly of the longitudinally opposite ends of the topsheet, and at least the undeformed portions of each of the elastic pieces are joined to the topsheet in the region where the longitudinally opposite ends of the core are situated. Each of the elastic pieces has at least the undeformed portions thermally bonded to the topsheet at a plurality of spaced apart sites in the region where the longitudinally opposite ends of the core are situated. By joining the elastic pieces to the topsheet in the region where the longitudinally opposite ends of the core are situated, the core having a relatively high rigidity is effectively utilized for shaping the absorbent article like a cup, and the topsheet and the backsheet are prevented from becoming locally bent at the corners of their front ends.

In a preferred embodiment of the invention, each of the elastic pieces is disposed above the topsheet such that the fold-back line defines the widthwise inward edge, each of the elastic pieces is joined to the topsheet at the widthwise outward edge and the longitudinally opposite ends of each of the elastic pieces, and the elastic pieces are caused to extend upwardly inclinedly in a widthwise inward direction at the longitudinally central portion of the absorbent article, owing to the contracting action of the elastic pieces. Each of the elastic pieces in an elastically elongated configuration includes. in the longitudinally central portion, an additional portion which extends further widthwise outwardly from the widthwise outward edge of the elastic piece joined to the topsheet, and which is then folded back to extend widthwise inwardly. The additional portion of the elastic piece may also be caused to extend upwardly inclinedly in a widthwise outward direction owing to the contracting action of the elastic pieces. The widthwise outward region of the additional portion of each of the elastic pieces can constitute a wing to be folded back onto the outside surface of panties, In another preferred embodiment of the invention, each of the elastic pieces is disposed such that the fold-back line defines the widthwise outward edge, each of the elastic pieces in an elastically elongated configuration is caused to extend widthwise outwardly beyond both side edges of the top sheet in the longitudinally central portion, each of the elastic pieces is joined to at least one of the topsheet and the backsheet at the widthwise outward edges and the longitudinally opposite ends of at least one of the topsheet and the backsheet. and the elastic pieces are caused to extend upwardly inclinedly in a widthwise outward direction in the longitudinally central portion, owing to the contracting action of the elastic pieces.

In the absorbent article constructed in accordance with one aspect of the present invention, each the elastic pieces extending upwardly on both sides of the longitudinally central portion of the absorbent article to form cuffs is formed from a web deformed at least partially in a non-planar configuration by machining. Such a web has a considerably decreased area of contact with the wearer's skin as compared with an entirely planar web which has not been machined. For this and other reasons, discomfort caused to the wearer, if any, is much less. Thus, the feeling of the absorbent article when worn is markedly improved over that of a conventional absorbent article. When a polyolefin thermoplastic film, such as a polyethylene or polyethylene blended film, is used as the web to be formed into the elastic piece by machining, the cost of the elastic piece as the material is sufficiently low. Moreover, the molecular structure of the topsheet and/or the backsheet and that of the elastic piece are rendered substantially the same or similar to each other, thereby enabling the thermal bonding of the elastic piece to the topsheet and/or the backsheet to be performed with ease. Thus, it becomes possible to lower the cost of manufacturing sufficiently and manufacture the absorbent article sufficiently easily.

### BRIEF DESCRIPTION OF THE DRAWINGS

While the specification concludes with claims particularly pointing out and distinctly claiming the present invention, it is believed that the present invention will be better understood from the following description in conjunction with the following drawings, in which like reference numbers identify identical elements and wherein:
Fig. 1 is a perspective view showing an embodiment of a sanitary napkin constructed in accordance with the present invention;
Fig. 2 is a plan view showing the sanitary napkin of Fig. 1 with the elastic pieces elongated to make the entire napkin flat.
Fig. 3 is a cross sectional view taken on line 3-3 of Fig. 1.
Fig. 4 is a plan view showing part of the web used preferably to form the elastic pieces of the sanitary napkin illustrated in Fig. 1.
Fig. 5 is a perspective view showing part of the web illustrated in Fig. 4.
Fig. 6 is a perspective view showing part of the web illustrated in Fig. 4 in an elastically somewhat elongated state.
Fig. 7 is a perspective view showing part of the web illustrated in Fig. 4 in a more elastically elongated state than the state of Fig. 6.
Fig. 8 is a diagram showing the relationship between a longitudinally exerted tensile force and elongation in the web illustrated in Fig. 4, and the relationship between a longitudinally exerted tensile force and elongation in an ordinary flat web before being converted into the web of Fig. 4 by machining.
Fig. 9 is a perspective view showing a processing means advantageousty used to form the web of Fig. 4.
Fig. 10 is a perspective view showing the web of Fig. 4 folded back along the undeformed portion.
Fig. 11 is a perspective view showing the web of Fig. 4 folded back along the deformed portion.
Fig. 12 is a perspective view showing another embodiment of a sanitary napkin constructed in accordance with the present invention.
Fig. 13 is a plan view, similar to Fig. 2, showing the sanitary napkin of Fig. 12 with the elastic pieces elongated to make the entire napkin flat.
Fig. 14 is a cross sectional view taken on line 14-14 of Fig. 12.
Fig. 15 is a perspective view showing still another embodiment of a sanitary napkin constructed in accordance with the present invention.
Fig. 16 is a plan view, similar to Fig. 2, showing the sanitary napkin of Fig. 15 with the elastic pieces elongated to make the entire napkin flat.
Fig. 17 is a cross sectional view taken on line 17-17 of Fig. 15.
Fig. 18 is a cross sectional view, similar to Fig. 3, showing a modified example of the sanitary napkin illustrated in Fig. 1.
Fig. 19 is a cross sectional view, similar to Fig. 3, showing a modified example of the sanitary napkin illustrated in Fig. 12.
Fig. 20 is a plan view, similar to Fig. 2, showing another modified example of the sanitary napkin illustrated in Fig. 1.
Fig. 21 is a cross sectional view, similar to Fig. 3, of the sanitary napkin illustrated in Fig. 20.
Fig. 22 is a cross sectional view, similar to Fig. 3, showing a modified example of the sanitary napkin illustrated in Fig. 20.
Fig. 23 is a plan view, similar to Fig. 2, showing still another modified example of the sanitary napkin illustrated in Fig. 1.
Fig. 24 is a cross sectional view, similar to Fig. 3, of the sanitary napkin illustrated in Fig. 23.
Fig. 25 is a plan view, similar to Fig. 2, showing a modified example of the sanitary napkin illustrated in Fig. 23.
Fig. 26 is a cross sectional view, similar to Fig. 3, of the sanitary napkin illustrated in Fig. 25.

### DETAILED DESCRIPTION OF THE INVENTION

Preferred embodiments of the present invention will be described in more detail with reference to the accompanying drawings.

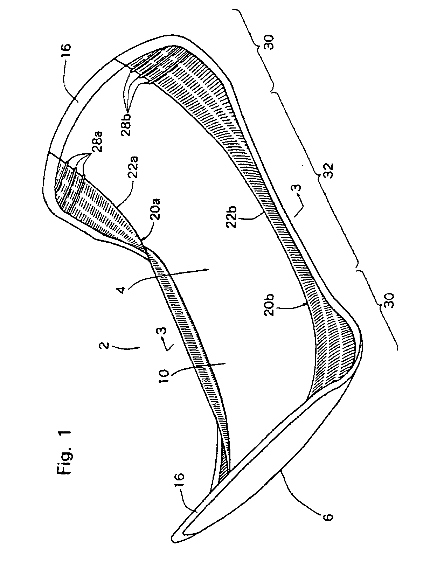

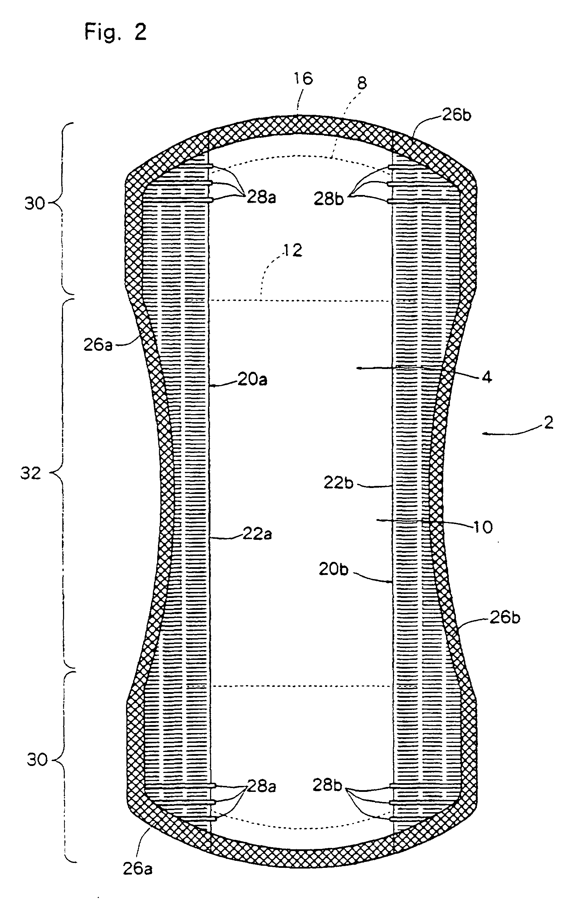

Figs. 1 to 3 show a sanitary napkin, an embodiment of an absorbent article constructed in accordance with the present invention. The napkin shown entirely at the numeral 2, as will be understood from the following description, is not in a substantial flat form, but is cup-shaped as illustrated in Fig. 1. owing to the contracting action of elastic pieces. In Fig. 2, the entire napkin 2 is shown in a substantially flat state with its elastic pieces elongated elastically. The napkin 2 indicated in Figs. 1 to 3 has a liquid pervious topsheet 4, a liquid impervious backsheet 6, and an absorbent core 8 disposed intermediate the topsheet 4 and the backsheet 6.

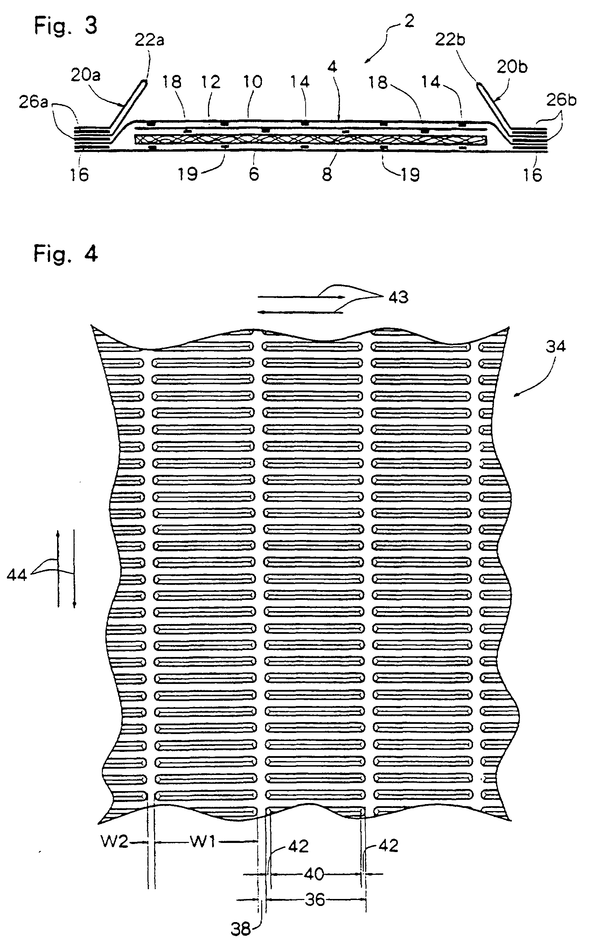

The topsheet 4 importantly should permit bodily discharges from the wearer to rapidly penetrate the absorbent core 8, and should not cause excessive discomfort to the wearer when it is contacted with the wearer's skin. The topsheet 4 can be formed advantageously from materials, such as woven or nonwoven fabrics of natural or synthetic fibers; apertured thermoplastic films; porous foams; reticulated foams; reticulated thermoplastic films; and thermoplastic scrims. In the illustrated embodiment, the topsheet 4 comprises a primary topsheet layer 10 and a secondary topsheet layer 12. In the state shown in Fig. 2, the primary topsheet layer 10 is nearly rectangular overall, with its longitudinally (up-and-down in Fig. 2) opposite edges extending convexly arcuately, and its opposite side edges extending straightly at the longitudinally opposite end portions, but extending concavely arcuately at the longitudinally central portion. Such primary topsheet layer 10 can be preferably formed from an apertured polyolefinic film as disclosed in the specification and drawings of U.S. Patents 3,929,135, 4,324,246, 4,342,314, 4,463,045 and 5,006,394. The secondary topsheet layer 12 is joined to the inner surface, i.e., undersurface, of the primary topsheet layer 10. The secondary topsheet layer 12 is rectangular, and its longitudinal and widthwise dimensions are smaller than the longitudinal and widthwise dimensions of the primary topsheet layer 10, with the peripheral edge portion of the primary topsheet layer 10 extending beyond the peripheral edge portion of the secondary topsheet layer 12. The secondary topsheet layer 12 desirably has the function to disperse a body fluid, which has passed through the primary topsheet layer 10, mainly in the longitudinal direction and cause it to flow into the absorbent core 8. Such secondary topsheet layer 12 can be formed preferably from a natural or synthetic nonwoven fabric. Particularly preferred nonwovens for forming the secondary topsheet layer 12 include a nonwoven fabric of spunbond polypropylene fibers designated P-9 available from the Fiberweb Corporation of Simpsonville, South Carolina, USA, under the tradename CELESTRA; and a nonwoven fabric formed of bicomponent fibers which have a polyethylene sheath and a polyestere core/a polyethylene sheath and a polypropylene core, the fabric available from the Havix Company, of Japan, as S2146. The primary topsheet layer 10 and the secondary topsheet layer 12 are advantageously joined together by adhesive bonding via a suitable adhesive, ultrasonic welding, or thermal bonding which is carried out in a multiplicity of discrete areas. In Fig. 3, the areas of bonding, welding or thermal bonding between the primary topsheet layer 10 and the secondary topsheet layer 12 are indicated by heavy solid lines for convenience of illustration.

The backsheet 6 importantly has the function to prevent body fluids absorbed to the absorbent core 8 from flowing out of the napkin and soiling the wearer and/or the wearer's clothing. The backsheet 6 can be advantageously formed from a flexible liquid impervious thermoplastic film such as a polyolefinic film. Particularly preferred films for forming the backsheet 6 include a low density polyethylene film 0.01 to 0.05 mm, particularly, about 0.025 mm, in thickness. Such a polyethylene film is sold by the Ethyl Corp., Visqueen Division, as Model XP-39385 and by the Clopay Corp. of Cincinnati, Ohio, USA, as SOFLEXX 1401. In the illustrated embodiment, the backsheet 6 has a shape and dimensions which are substantially the same as the shape and dimensions of the primary topsheet layer 10 of the topsheet 4. The primary topsheet layer 10 of the topsheet 4 and the backsheet 6 are thermally bonded uninterruptedly throughout their peripheral edges. For convenience of illustration, the area 16 of thermal bonding between the primary topsheet layer 10 and the backsheet 6 is represented by intersecting diagonal lines in Fig. 2, and heavy solid lines in Fig. 3. If desired, the primary topsheet layer 10 and the backsheet 6 may be joined together in other suitable manner, such as bonding with an adhesive. Where necessary, a pressure sensitive adhesive for detachably securing the napkin 2 to required sites on the inner surface of the undergarment of the wearer can be applied to the outside surface, i.e., the undersurface, of the backsheet 6 in one or more longitudinally extending bands. A suitable pressure sensitive adhesive is Century Adhesive A-305-IV sold by Century Adhesive Corp. of Columbus, Ohio, USA. In applying the pressure sensitive adhesive to the underside of the backsheet 6, it is desirable to cover the pressure sensitive adhesive with a silicone coated paper in order to prevent its soiling prior to use.

Figs. 1 to 3, especially Fig. 3, will be referred to for further explanation. The absorbent core 8 disposed between the topsheet 4 and the backsheet 6 importantly functions to absorb and retain body fluids that have penetrated the topsheet 4 after discharge from the wearer. Such absorbent core 8 can be advantageously formed from materials, such as comminuted wood pulp called airfelt; creped cellulose wadding; meltblown polymers; chemically stiffened, modified, or crosslinked cellulosic fibers, absorbent foams; layers of tissue paper; absorbent gelling materials (materials gelling when absorbing liquids); or any suitable combinations of these. The longitudinal and widthwise dimensions of the illustrated core 8 are smaller than the longitudinal and widthwise dimensions of the primary topsheet layer 10 and the backsheet 6, with the core 8 being situated inwardly of the areas 16 of thermal bonding between the primary topsheet layer 10 and the backsheet 6. Advantageously, the longitudinal and widthwise dimensions of the secondary topsheet layer 12 in the topsheet 4 are somewhat smaller than or substantially equal to the longitudinal and widthwise dimensions of the core 8. The top surface of the core 8 is joined to the undersurface of the secondary topsheet layer 12, while the undersurface of the core 8 is joined to the inner surface or top surface of the backsheet 6. The joining of the core 8 and the secondary topsheet layer 12 and the joining of the core 8 and the backsheet 6 are advantageously achieved by bonding via a suitable adhesive which is carried out in a multiplicity of discrete areas. If desired, the core 8 may be joined to the secondary topsheet layer 12 and/or the backsheet 6 in other suitable manner, such as ultrasonic welding or thermal bonding. In Fig. 3, the discrete areas 18 of bonding, welding or thermal bonding between the core 8 and the secondary topsheet layer 12, and the discrete areas 19 of bonding, welding or thermal bonding between the core 8 and the backsheet 6 are indicated by heavy solid lines for convenience of illustration.

With further reference to Figs. 1 to 3, the illustrated napkin 2 constructed in accordance with the present invention further includes a pair of band-like elastic pieces 20a and 20b. Each of the band-like elastic pieces 20a and 20b is situated above the topsheet 4 in an elastically elongated state, and is joined to the topsheet 4 at required sites. As will be easily understood by reference to Fig. 3, the respective elastic pieces 20a and 20b are folded back along longitudinally extending fold-back lines 22a and 22b to have a two-layer configuration, and are arranged on both sides of the topsheet 4, with the fold-back lines 22a and 22b located widthwise inwardly In the state illustrated in Fig. 2 in which the elastic pieces 20a and 20b are elongated to make the entire napkin 2 flat, the widthwise inward edges of the elastic pieces 20a and 20b, i.e., the fold-back lines 22a and 22b, extend substantially straightly in the longitudinal direction. On the other hand, the widthwise outward edge and longitudinally opposite edges of each of the elastic pieces 22a and 22b are rendered consistent with the widthwise outward edge and opposite edges of the topsheet 4. Each of the elastic pieces 20a and 20b has the respective layers of the two-layer structure thereof thermally bonded together, and is thermally bonded to the primary topsheet layer 10 of the topsheet 4, at the widthwise outward edge and longitudinally opposite edges thereof. Such areas 26a and 26b of thermal bonding are represented by intersecting diagonal lines in Fig. 2, and heavy solid lines in Fig. 3, for convenience of illustration. As one will easily understand by referring to Figs. 2 and 3, the areas 26a and 26b of thermal bonding are substantially conformed to the main part of the above-mentioned area 16 of thermal bonding between the primary topsheet layer 10 of the topsheet 4 and the backsheet 6 (i.e., that part in the area 16 of heating which excludes the widthwise central area of the longitudinally opposite edges where the elastic pieces 20a and 20b are not present). Furthermore, each of the elastic pieces 20a and 20b has the respective layers of the two-layer structure thereof thermally bonded together, and is thermally bonded to the primary topsheet layer 10 of the topsheet 4, at a plurality of longitudinally spaced apart sites in each of the longitudinally opposite end portions, and more specifically, at three sites 28a and 28b of thermal bonding delineated in solid lines in Figs. 1 and 2, by operating a thermal bonding tool (not shown) there. importantly, at least part of the plurality of thermal bonding sites 28a and 28b is present within the region where the longitudinally opposite end portions of the core 8 are located below the primary topsheet layer 10 of the topsheet 4. In the illustrated embodiment, as will be understood from Fig. 2, one thermal bonding site 28a and one thermal bonding site 28b located at the outermost position In each of the longitudinally opposite end portions of the elastic pieces 20a and 20b are situated outwardly of the longitudinal end of the core 8 Whereas the remaining two thermal bonding sites 28a and 28b are located Inwardly of the longitudinal end of the core 8. If desired, instead of thermal bonding, other suitable method may be employed to join the elastic pieces 20a and 20b to the primary topsheet layer 10 of the topsheet 4 (where necessary, while joining the respective layers of their two-layer structure to each other).

As stated above, the elastic pieces 20a and 20b are located above the topsheet 4 in an elongated state, and are thermally bonded thereto at the areas 26a and 26b of thermal bonding as well as the sites 28a and 28b of thermal bonding. Afterwards, the force that has maintained the elastic pieces 20a and 20b in an elongated condition is released. As a result, the non-thermally bonded portions of the elastic pieces 20a and 20b elastically contract, and thus, the elastic pieces 20a and 20b try to restore their original lengths at least partially. Owing to this contracting action generated in the elastic pieces 20a and 20b, the topsheet 4, the backsheet 6 and the core 8 are displaced to extend upwardly inclinedly toward the longitudinally opposite ends in each of the longitudinally opposite end portions 30, as clearly illustrated in Fig. 1. Thus, the napkin 2 is brought into the shape of a cup as a whole. In addition, in the longitudinally central portion 32, each of the elastic pieces 20a and 20b is caused to extend upwardly inclinedly in a widthwise inward direction (accordingly, toward the aforementioned fold-back lines 22a and 22b). Thus, on both sides of the longitudinally central portion 32 of the napkin 2, the elastic pieces 20a and 20b form cuffs which function as barriers for preventing body fluids from leaking widthwise outwardly. The elongation of the elastic pieces 20a and 20b during thermal bonding depends on the entire configuration and dimensions of the napkin Itself. Normally, however, it is preferred to set the entire length at elongation at about 110 to 160%. especially, about 115 to 145%, of the entire length at non-elongation.

According to our experience, if thermal bonding is not performed at the sites 28a and 28b of thermal bonding, the primary topsheet layer 10 of the topsheet 4 and the backsheet 6 tend to be locally bent at the four corners of the napkin 2 owing to the contracting action of the elastic pieces 20a and 20b, since the primary topsheet layer 10 of the topsheet 4 and the backsheet 6 have relatively low stiffness. When thermal bonding is performed at the sites 28a and 28b of thermal bonding, on the other hand, the elastically contracting action of the elastic pieces 20a and 20b is locally eliminated between the sites 28a and 28b of thermal bonding. Also, the elastic pieces 20a and 20b are locally joined to the relatively highly stiff core 8 via the primary topsheet layer 10, whereby the contracting action of the elastic pieces 20a and 20b is effectively transmitted to the core 8. Consequently, the undesirable local bending of the primary topsheet layer 10 of the topsheet 4 and the backsheet 6 at the four corners of the napkin 2 is prevented, and the topsheet 4, backsheet 6 and core 8 are caused to extend upwardly inclinedly toward the longitudinally opposite ends, as desired, in each of the longitudinally opposite end portions 30.

In the napkin 2 constituted in accordance with the present invention, the elastic pieces 20a and 20b are formed from a web deformed at least partially in a non-planar configuration by machining. The web consists in a polyolefinic thermoplastic film, such as a polyethylene film, which includes, in a suitable combination, deformed portions deformed in a non-planar configuration by machining, and undeformed portions retained in a planar configuration. Preferably, the undeformed portions extend longitudinally in a continuous or discrete manner. When the elastic pieces 20a and 20b are formed from a web including the deformed portions and the longitudinally extending undeformed portions, the contracting action of the elastic pieces 20a and 20b joined in an elastically elongated state to the topsheet 4 is generated mainly by the elastic restoring effect of the undeformed portions, as will be clearly understood from the description to be given below.

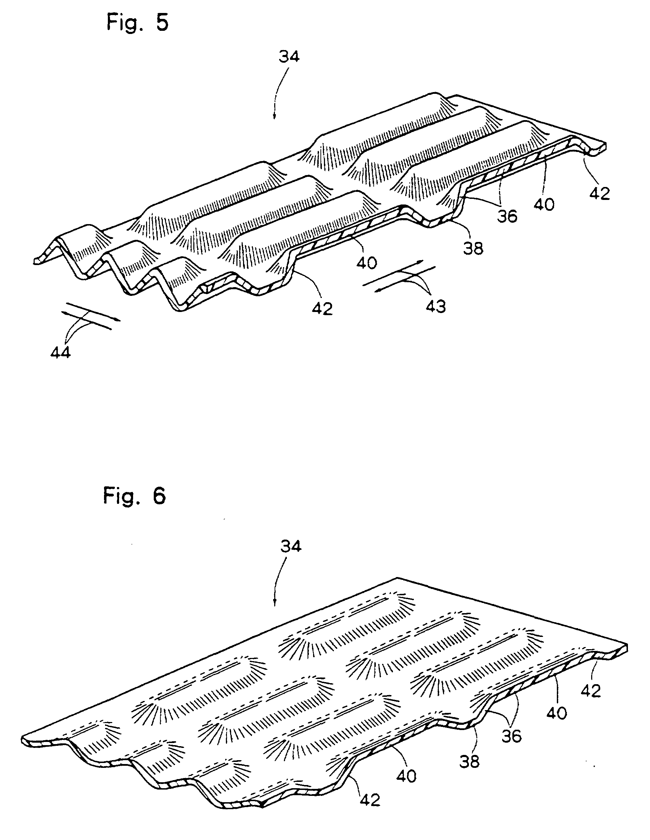

Figs. 4 and 5 show a web 34 that can be used preferably for forming the elastic pieces 20a and 20b. This web 34 is composed of deformed portions 36 deformed in a non-planar configuration by machining, and undeformed portions 38 retained in a planar configuration. As will be seen clearly from Fig. 4, the deformed portions 36 and the undeformed portions 38 are arranged alternately in a side-by-side relationship in the widthwise direction shown by arrows 43 (in the right-and-left direction in Fig. 4), and are caused to extend uninterruptedly in the longitudinal direction shown by arrows 44 (in the up-and-down direction in Fig. 4) As will be understood by reference to Fig. 5, the deformed portion 36 includes a primary deformed portion 40, and secondary deformed portions 42 located on both sides in its widthwise direction. The primary deformed portion 40 takes a substantially uniform nearly wavy form in its longitudinal cross section, and takes a substantially horizontally extending straight form in its widthwise cross section. The secondary deformed portion 42 is a region of transition from the primary deformed portion 40 and the undeformed portion 38. The proportion of the undeformed portions 38 in the web 34 is about 1 to 30%, preferably 3 to 20%, more preferably 5 to 12%, when calculated as the rate of area on the assumption that the web 34 is in a non-elongated state and the deformed portions 36 (including the primary deformed portions 40 and the secondary deformed portions 42) are in an undeformed planar configuration, that is, the state illustrated as such in Fig. 4 (a non-elongated plan view). In other words, the proportion of the deformed portions 36 (including the primary deformed portions 40 and the secondary deformed portions 42) in the web 34 is about 70 to 99%, preferably 80 to 97%, more preferably 88 to 95%, when calculated as the rate of area on the assumption that the web 34 is in a non-elongated state and the deformed portions 36 (including the primary deformed portions 40 and the secondary deformed portions 42) are in an undeformed planar configuration, that is, the state illustrated as such in Fig. 4 (a non-elongated plan view). If the proportion of the undeformed portions 38 in the web 36 is too high, the elastic elongation of the elastic pieces 20a and 20b tends to become relatively difficult. If the proportion of the undeformed portions 38 in the web 36 is too low, the contracting action of the elastic pieces 20a and 20b joined in an elastically elongated state to the topsheet 4 is minimal, making it difficult to shape the napkin 2 into a desired cup-like form. Furthermore, the following fact should also be noticed in regard to the proportion of the undeformed portions 38 in the web 36: Since the deformed portions 36 are in a non-planar configuration, they touch the skin of the napkin 2 wearer only locally at a plurality of small spaced apart regions. Consequently, discomfort they cause to the wear is limited, if any In contrast, if the proportion of the undeformed portions 38 is too high and the undeformed portions 38 are the main source of contact with the skin of the napkin wearer, the undeformed portions 38 in a planar configuration touch the wearers skin uninterruptedly over a relatively large area. Thus, they cause considerable discomfort to the wearer. Generally, the width W1 of the deformed portion 36 is 0.25 to 50.80 mm (0.01 to 2.00 inches), preferably, 3.18 to 25.40 mm (0.13 to 1.00 inch), while the width W2 of the undeformed portion 38 is 0.25 to 12.70 mm (0.01 to 0.50 inch), preferably, 0.76 to 6.35 mm (0.03 to 0.25 inch).

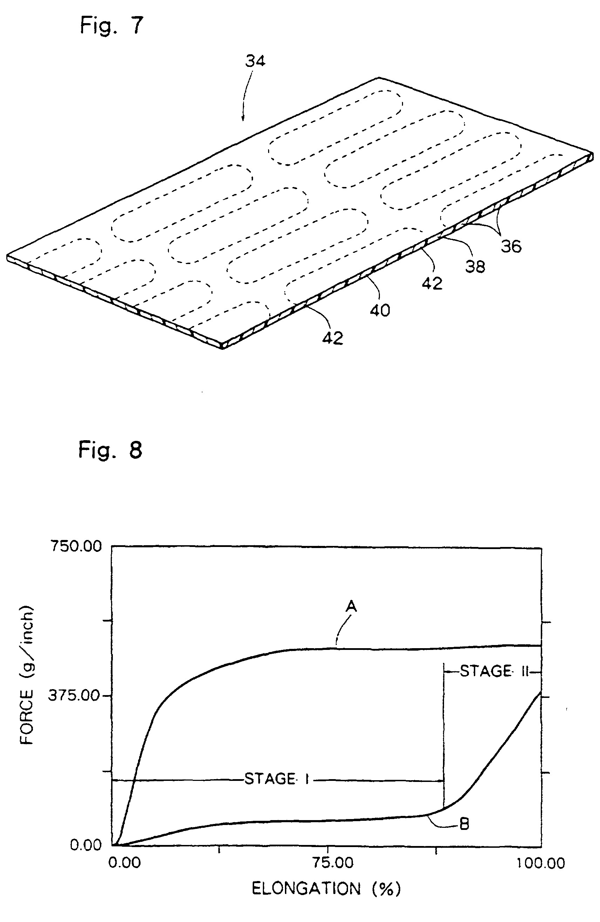

The web 34 shown in Figs. 4 and 5 exhibits desired elasticity as the elastic pieces 20a and 20b in the longitudinal direction indicated by arrows 44. When a longitudinal tensile force is exerted on the web 34 to elongate it, its cross sectional configuration is geometrically changed as illustrated in Fig. 6, with its wavy ups and downs being gradually decreased. Upon a further elongation, the wavy configuration substantially vanishes as shown in Fig. 7. During the change of the deformed portions 36 from the state illustrated in Fig. 5 to the state in Fig. 6, and finally to the state in Fig. 7, the resistive force of the deformed portions 36 to the elongation is markedly low. The undeformed portions 38, on the other hand, are elongated by the molecular-level deformation of the constituent material itself, and the resistive force of the web 34 to the elongation is mainly the resistive force of the undeformed portions 38. In other words, in the range where the elongation of the deformed portions 36 is attributable to geometric deformation, rather than to the molecular-level deformation of the material itself, the contracting action of the web 34 upon release of the tensile force exerted on the web 34 results mainly from the contracting action of the undeformed portions 38 elongated due to the molecular-level deformation of the material per se.

A more detailed description of the elongation and contraction of the web 34 will be offered. Fig. 8 shows the relationship between a longitudinally exerted tensile force and elongation in a specific web. Curve A depicts the results of measurements of a longitudinally exerted tensile force and elongation in an ordinary web consisting entirely of the undeformed portions Curve B reveals the measurement results on the relation between a longitudinally exerted tensile force and elongation in a web machined to have the form illustrated in Figs. 4 and 5. In the measurements that gave the results represented by curve A, the web comprised entirely of the undeformed web was a linear low density polyethylene film, approximately 0.025 mm in thickness, designated sample 1401 available from Clopay Corp., Cincinnati, Ohio, U.S.A. in the measurements that gave the results represented by curve B, a web comprising the same linear low density polyethylene film subjected to predetermined machining was used As will be understood from curve B, the web 34 made into the form of Figs. 4 and 5 by machining has a stage I with a relatively low tensile force for a relatively high elongation, and a stage II with a sharply increased tensile force for elongation. In the stage I elongation range, release of the tensile force results in fully satisfactory elastic contraction. When the web 34 is to be used as the elastic pieces 20a and 20b in the napkin 2 illustrated in Figs. 1 to 3, it is important that the elongation of the web 34 be maintained in the stage I region. Suitable adjustments can be made by varying the shape of curve B in Fig. 8, i.e., the relation between the longitudinally exerted tensile force and the elongation in the starting web 34, the proportions of the deformed portions 36 and the undeformed portions 38 in the web 34, and so on.

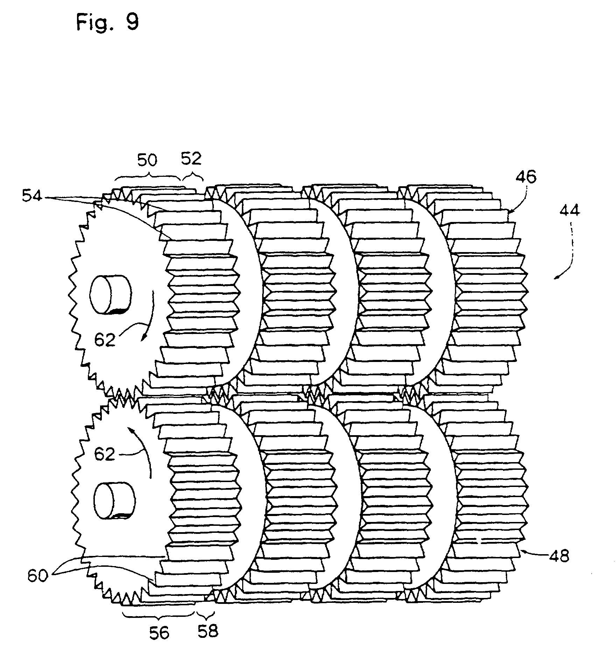

Fig. 9 shows a processing means 44 for preferable use in applying desired machining. The processing means 44 includes a pair of rotationally processing rollers 46 and 48 acting cooperatively. The processing roller 46 has processing regions 50 and non-processing regions 52 arranged alternately in the widthwise direction, the processing region 50 having a multiplicity of processing projections 54 all over its circumference. Likewise, the processing roller 48 has processing regions 56 and non-processing regions 58 arranged alternately in the widthwise direction, the processing region 56 having a multiplicity of processing projections 60 all over its circumference. The processing regions 50 of the processing roller 46 and the processing regions 56 of the processing roller 48 are located at corresponding positions, and the processing projections 54 and the processing projections 60 work cooperatively. The non-processing regions 52 of the processing roller 46 are positioned in correspondence with the non-processing regions 58 of the processing roller 48. When an ordinary unprocessed web is fed between the processing rollers 46 and 48 continuously rotated in the directions shown by arrows 62, the portions of the web passing between the processing regions 50 and 56 of the processing rollers 46 and 48 are machined, producing the deformed portions 36. The portions of the web passing between the non-processing regions 52 and 58 of the processing rollers 46 and 48 are not machined, but left in a substantially planar configuration, constituting the undeformed portions 38. Suitable web materials to be partially machined for making a web including the deformed portions 36 and the undeformed portions 38 are polyolefin thermoplastic films including linear low density polyethylene (LLDPE), low density polyethylene (LDPE), ultra low density polyethylene (ULDPE), high density polyethylene (HDPE), polypropylene, or blends of these. If desired, web materials to be partially machined for making a web including the deformed portions 36 and the undeformed portions 38 may be polyester, polyurethane, compostable or biodegradable polymers, heat shrink polymers, thermoplastic elastomers, metallocene catalyst-based polymers (e.g, INSITE available from Dow Chemical Company, and Exxact available from Exxon), and breathable polymers.

In the web 34 described with reference to Figs. 4 to 9, the deformed portions 36 and the undeformed portions 38 are caused to extend longitudinally continuously. If desired, the undeformed portions extending longitudinally over a predetermined length may be suitably arranged discretely at suitably spaced apart locations. Also, instead of causing the deformed portions and/or undeformed portions to extend substantially straightly in the longitudinal direction, it is possible to cause these portions to extend inclinedly in a suitable direction, or with suitable curvature.

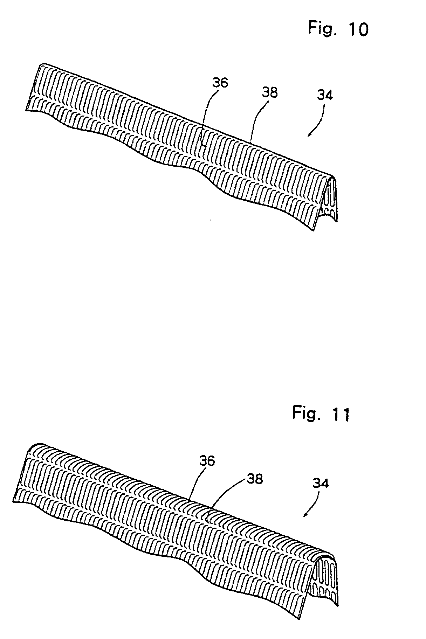

In the napkin 2 illustrated in Figs. 1 to 3, as has been described, the elastic pieces 20a and 20b are folded back along the fold-back lines 22a and 22b to have a two-layer configuration. If the elastic pieces 20a and 20b are formed from the web 34 explained by reference to Figs. 4 to 9, the fold-back lines 22a and 22b can be caused to extend along the undeformed portion 38 as shown in Fig. 10, or the fold-back lines 22a and 22b can be caused to extend along the deformed portion 36 as shown in Fig. 11. When the fold-back lines 22a and 22b are caused to extend along the undeformed portion 38, the upper edge of the fold becomes relatively sharp, providing a good contact with the wearer's skin. When the fold-back lines 22a and 22b are caused to extend along the deformed portion 36, the fold-back line becomes relatively dull, improving the feeling of the napkin when worn.

In forming the elastic pieces 20a and 20b from the web 36 described by reference to Figs. 4 to 9, the following facts should also be noticed: As have been mentioned referring to Figs. 1 to 3, the elastic pieces 20a and 20b are thermally bonded to the topsheet 4 at the plurality of sites 28a and 28b of thermal bonding as well as the regions 26a and 26b of thermal bonding. Since the contracting action of the elastic pieces 20a and 20b is generated mainly by the undeformed portions 38 as aforementioned, it is important that the sites 28a and 28b of thermal bonding include the undeformed portions 38. If thermal bonding is not limited to the undeformed portions 38, but partially extends to the deformed portions 36 as well, the mechanical deformation of the deformed portions 36 is destroyed by the thermal bonding. If such spoiled sites touch the wearers skin, they irritate it Therefore, the areas of the sites 28a and 28b of thermal bonding should preferably be minimized. It is also preferred that a plurality of the sites 28a and 28b of thermal bonding of a relatively small area be disposed at spaced apart locations instead of providing one site 28a and one site 28b of thermal bonding of a relatively large area.

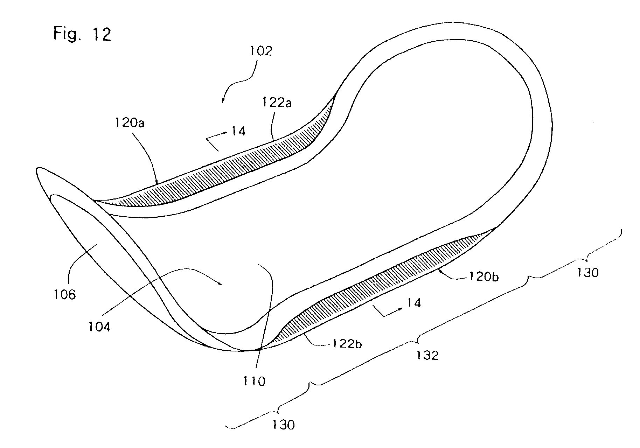

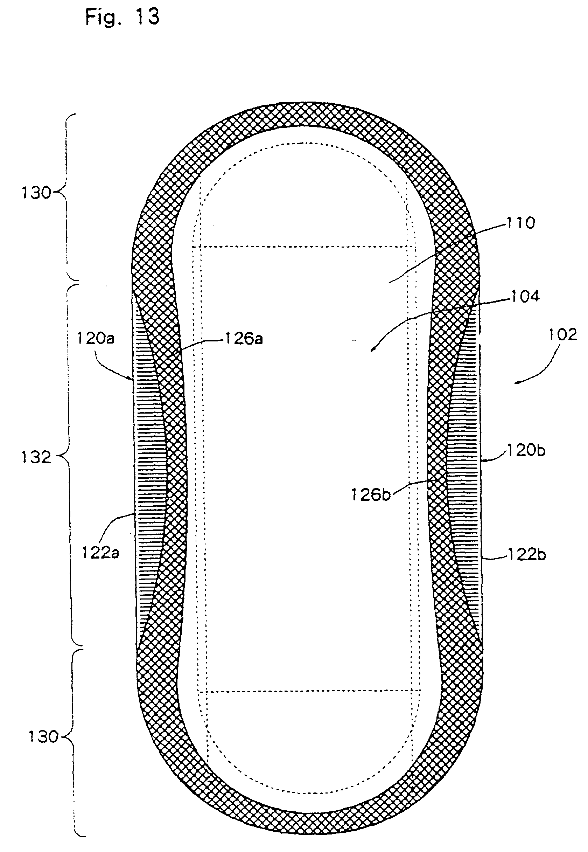

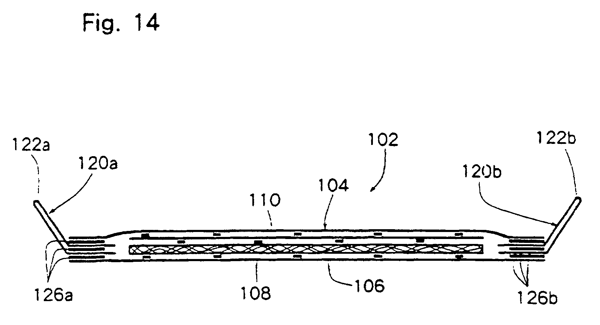

Figs. 12 to 14 show a sanitary napkin, another embodiment of the absorbent article constituted in accordance with the present invention. In the napkin indicated entirely at 102, the radius of curvature of a convex arc defined by each of the longitudinally opposite edges is set to be relatively small, and the longitudinally opposite edges each take a nearly semicircular form. A pair of band-like elastic pieces 120a and 120b in the napkin 102 are each folded back along longitudinally extending fold-back edges 122a and 122b to have a two-layer configuration. These elastic pieces 120a and 120b are arranged on both sides of the napkin 102 such that the fold-back edges 122a and 122b are located widthwise outwardly, in other words, such that the fold-back edges 122a and 122b define the widthwise outward edges. In the state illustrated in Fig. 13 in which the elastic pieces 122a and 122b are elongated to make the entire napkin 102 flat, the side edges of a topsheet 104 and a backsheet 106 in a longitudinally central portion 132 extend in a concavely arcuate form. On the other hand, the widthwise outward edges 122a and 122b of the elastic pieces 122a and 122b are caused to extend substantially straightly even in the longitudinally central portion 132. In the longitudinally central portion 132, the elastic pieces 122a and 122b are caused to extend widthwise outwardly beyond the topsheet 104 and the backsheet 106. As will be clearly understood from Fig. 14, each of the elastic pieces 122a and 122b is disposed between the topsheet 104 and the backsheet 106 on both sides of the napkin 102. At the areas 126a and 126b of thermal bonding (represented by intersecting diagonal lines in Fig. 13, and heavy solid lines in Fig. 14) extending along the widthwise outward edges and the longitudinally opposite edges of the topsheet 104 and the backsheet 106, the elastic pieces 120a and 120b each have the respective layers of their two-layer structure thermally bonded together, and are thermally bonded to the primary topsheet layer 110 of the topsheet 104 and the backsheet 106.

The elastic pieces 120a and 120b are thermally bonded, in an elongated state as illustrated in Fig. 13, to the topsheet 104 (more specifically its primary topsheet layer 110) and the backsheet 106. After the thermal bonding, the force that has kept the elastic pieces 120a and 120b in an elongated condition is released. As a result, the non-thermally bonded portions of the elastic pieces 120a and 120b, i.e., the portions caused to extend widthwise outwardly beyond the side edges of the topsheet 104 and the backsheet 106 in the longitudinally central portion 132, and the portions situated inwardly of the areas 126a and 126b of thermal bonding in the widthwise and the longitudinal direction, elastically contract, and the elastic pieces 120a and 120b try to restore their original lengths at least partially. Owing to this contracting action generated in the elastic pieces 120a and 120b, the topsheet 104, the backsheet 106 and the core 108 are displaced to extend upwardly inclinedly toward the longitudinally opposite ends in each of the longitudinally opposite end portions 130, as clearly illustrated in Fig. 12. Thus, the napkin 102 is brought into the shape of a cup as a whole In addition, in the longitudinally central portion 132, each of the elastic pieces 120a and 120b is caused to extend upwardly inclinedly in a widthwise outward direction (accordingly, toward the aforementioned fold-back lines 122a and 122b). Thus, on both sides of the longitudinally central portion 132 of the napkin 102, the elastic pieces 120a and 120b form cuffs which function as barriers for preventing body fluids from leaking widthwise outwardly.

That constitution of the napkin 102 illustrated in Figs. 12 to 14 which is other than that described above may be substantially the same as the constitution of the napkin 2 shown in Figs. 1 to 3. Its explanation will be omitted herein to avoid overlapping.

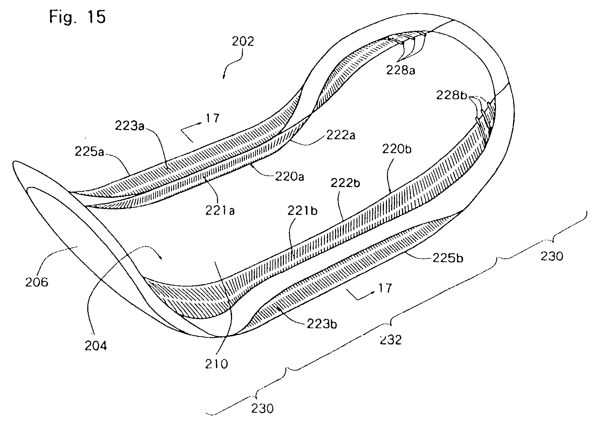

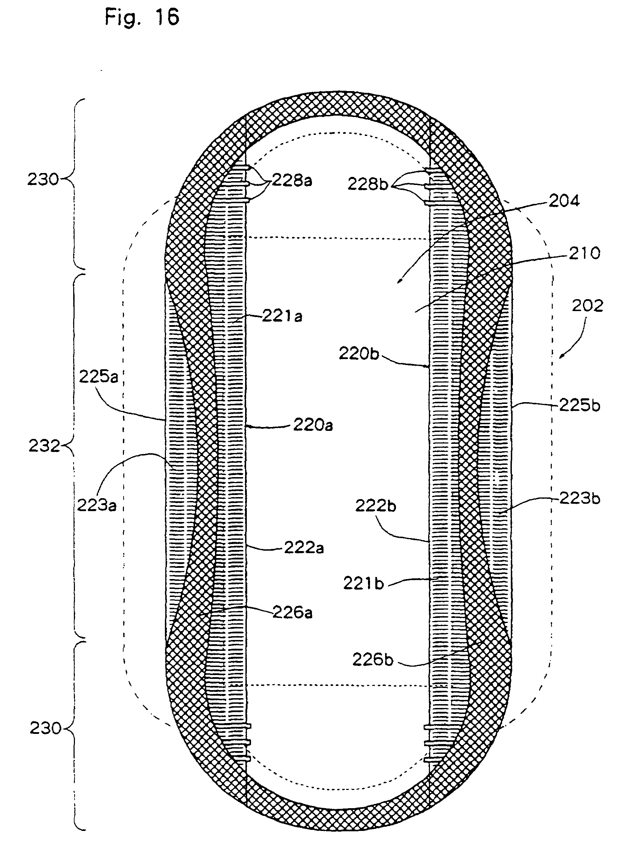

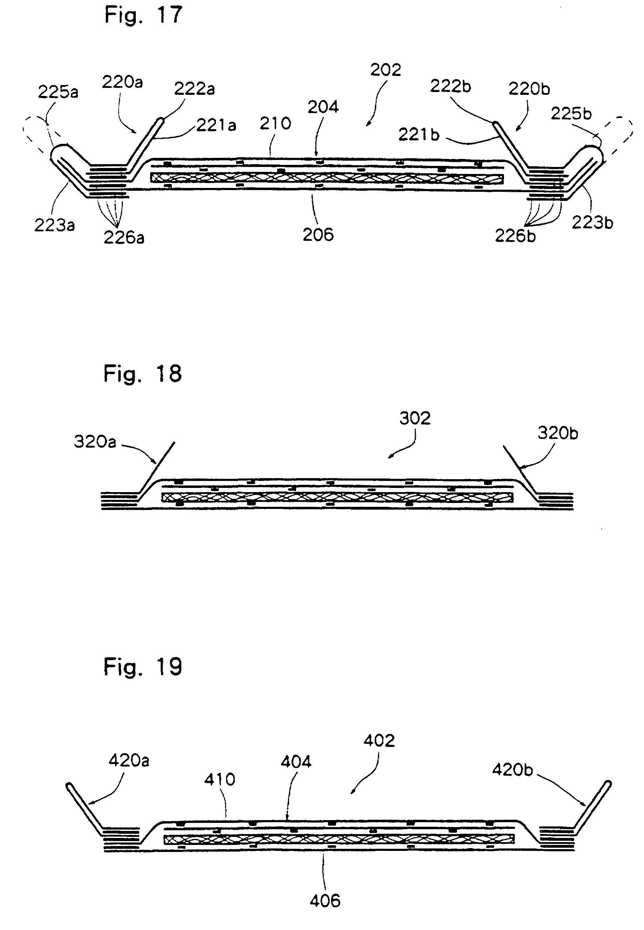

Figs. 15 to 17 show a sanitary napkin, still another embodiment of the absorbent article constituted in accordance with the present invention. In the napkin indicated entirely at 202, too, the radius of curvature of a convex arc defined by each of the longitudinally opposite edges is set to be relatively small, and the longitudinally opposite edges each take a nearly semicircular form. The napkin 202 includes a pair of band-like elastic pieces 220a and 220b. Each of the pair of band-like elastic pieces 220a and 220b has main portions 221 a and 221 b and additional portions 223a and 223b. The main portions 221 a and 221 b of the elastic pieces 220a and 220b are substantially the same as the elastic pieces 20a and 20b in the napkin 2 illustrated in Figs. 1 to 3, and are folded back along fold-back lines 222a and 222 to have a two-layer configuration. These main portions 221 a and 221 b are arranged on both sides of the topsheet 4, with the fold-back lines 222a and 222 being located widthwise inwardly. In the state illustrated in Fig. 16 in which the elastic pieces 220a and 220b are elongated to make the entire napkin 202 flat, the widthwise inward edges of the respective main portions 221a and 221b of the elastic pieces 220a and 220b, i.e , the fold-back lines 222a and 222b, extend substantially straightly in the longitudinal direction. The respective additional portions 223a and 223b of the elastic pieces 220a and 220b are caused to extend as a continuation from the widthwise outward edge of the upper layer of the main portions 221a and 221 b, and are folded back along additional fold-back lines 225a and 225b to have a two-layer configuration. The additional fold-back lines 225a and 225b each defining the widthwise outward edge of the elastic pieces 221 a and 221 b extend substantially straightly in the state illustrated in Fig. 16 in which the elastic pieces 220a and 220b are elongated to make the entire napkin 202 flat. As will be understood clearly from Fig 17, the widthwise inward portion of the lower layer of each of the respective additional portions 223a and 223b of the elastic pieces 220a and 220b is located downwardly of backsheet 206. The elastic pieces 220a and 220b having the main portions 221a and 221 b and the additional portions 223a and 223b each have the respective layers of their two-layer structure thermally bonded together directly and/or via the topsheet 204 (more specifically, its primary topsheet layer 210) and the backsheet 206, and are thermally bonded to the topsheet 204 and the backsheet 206, at the areas 226a and 226b of thermal bonding (represented by intersecting diagonal lines in Fig. 16, and heavy lines in Fig. 17). As will be seen unequivocally by comparative reference to Figs. 16 and 13, the areas 226a and 226b of thermal bonding in the napkin 202 shown in Figs. 15 to 17 are substantially the same in configuration as the areas 126a and 126b of thermal bonding in the napkin 102 shown in Figs 12 to 14 As clearly illustrated in Figs. 16 and 17, in the longitudinally central portion 232 of the napkin 202, the additional portions 223a and 223b of the elastic pieces 220a and 220b are caused to extend widthwise outwardly beyond the areas 226a and 226b of thermal bonding. As seen from Fig. 17, both side edges of the primary topsheet layer 210 of the topsheet 204 and the backsheet 206 are caused to extend substantially vertically in the state illustrated in Fig. 16 in which the elastic pieces 220a and 220b are elongated to make the entire napkin 202 flat. Thus, in the longitudinally central portion 232 of the napkin 202, the primary topsheet layer 210 of the topsheet 204 and the backsheet 206 are caused to extend widthwise outwardly beyond the areas 226a and 226b of thermal bonding, and are thereby located between the two layers of the additional portions 223a and 223b of the elastic pieces 220a and 220b. From the viewpoint of savings in the amounts of the materials used for the primary topsheet layer 210 of the topsheet 204 and the backsheet 206, both side edges of the primary topsheet layer 210 of the topsheet 204 and the backsheet 206 may, if desired, be substantially conformed to, or slightly inward of, the outward edges of the areas 226a and 226b of thermal bonding. As clearly shown in Figs. 15 and 16, each of the main portions 221 a and 221 b of the elastic pieces 220a and 220b, like the elastic pieces 20a and 20b in the napkin 2 shown in Figs. 1 to 3, has the respective layers of the two-layer structure thereof thermally bonded together, and is thermally bonded to the primary topsheet layer 210 of the topsheet 204, at a plurality of spaced apart sites, and more specifically at three sites 228a and 228b of thermal bonding delineated in solid lines in Figs. 15 and 16, by operating a thermal bonding tool (not shown) there.

In the napkin 202 shown in Figs. 15 to 17 as well, the elastic pieces 220a and 220b are thermally bonded, in an elongated state as illustrated in Fig. 16, to the topsheet 204 (more specifically its primary topsheet layer 210) and the backsheet 206 After such thermal bonding, the force that has kept the elastic pieces 220a and 220b elongated is released. By so doing, the non-thermally bonded portions of the elastic pieces 220a and 220b, i.e., the portions situated inwardly and outwardly of the areas 226a and 226b of thermal bonding (excluding the sites 228a and 228b of thermal bonding), elastically contract, and the elastic pieces 220a and 220b try to restore their original lengths at least partially. Owing to this contracting action generated in the elastic pieces 220a and 220b, the topsheet 204, the backsheet 206 and the core 208 are displaced to extend upwardly inclinedly toward the longitudinally opposite ends in each of the longitudinally opposite end portions 230, as clearly illustrated in Fig. 15. Thus, the napkin 202 is brought into the shape of a cup as a whole. In addition, in the longitudinally central portion 232, the respective main portions 221a and 221b of the elastic pieces 220a and 220b are caused to extend upwardly inclinedly in a widthwise inward direction (accordingly, toward the aforementioned fold-back lines 222a and 122b). Thus, the main portions 221a and 221b of the elastic pieces 220a and 220b form cuffs which function as barriers for preventing body fluids from leaking widthwise outwardly. The additional portions 223a and 223b of the elastic pieces 220a and 220b are also caused to extend upwardly inclinedly in a widthwise outward direction (accordingly, toward the aforementioned additional fold-back lines 225a and 225b). Thus, the additional portions 223a and 223b of the elastic pieces 220a and 220b form cuffs which function as barriers for preventing body fluids from leaking widthwise outwardly.

That constitution of the napkin 202 illustrated in Figs. 15 to 17 which is other than that described above may be substantially the same as the constitution of the napkin 2 shown in Figs. 1 to 3 and/or the napkin 102 shown in Figs. 12 to 14. Its explanation will be omitted herein to avoid overlapping.

Instead of constituting the additional cuffs by the additional portions 223a and 223b of the elastic pieces 220a and 220b, the widthwise outwardly protruding lengths of the additional portions 223a and 223b of the elastic pieces 220a and 220b, particularly, in the longitudinal central portion 232 of the napkin 202, may, if desired, be made relatively large as illustrated by two-dot chain lines in Figs. 16 and 17 so that the widthwise outward regions of the additional portions 223a and 223b can be used as wings to be folded back onto the outside surface of panties. In this case, a pressure sensitive adhesive may be applied, where necessary, to the undersurface of the widthwise outward regions of the additional portions 223a and 223b (the surface to be contacted with the outside surface of panties when these regions are folded back). Even without a pressure sensitive adhesive applied, when the additional portions 223a and 223b are folded back onto the outside surface of panties, these portions 223a and 223b are kept in fully intimate contact with the outside surface of the panties because of the elasticity of these portions 223a and 223b, whereby the napkin 202 can be set in place relative to the panties.

Fig. 18 shows a modified example of the napkin 2 illustrated in Figs. 1 to 3. In a napkin 302 as shown in Fig. 18, each of a pair of elastic pieces 320a and 320b is formed in a single-layer configuration rather than a two-layer configuration. The other points of constitution for the napkin 302 are substantially the same as the constitution of the napkin 2 depicted in Figs. 1 to 3.

Fig 19 shows a modified example of the napkin 102 illustrated in Figs. 12 to 14 In a napkin 402 as shown in Fig. 19, widthwise inward portions of each of a pair of elastic pieces 420a and 420b are positioned not between a primary topsheet layer 410 of a topsheet 404 and a backsheet 406, but above the primary topsheet layer 410 of the topsheet 404. The other points of constitution for the napkin 402 are substantially the same as the constitution of the napkin 2 depicted in Figs. 1 to 3

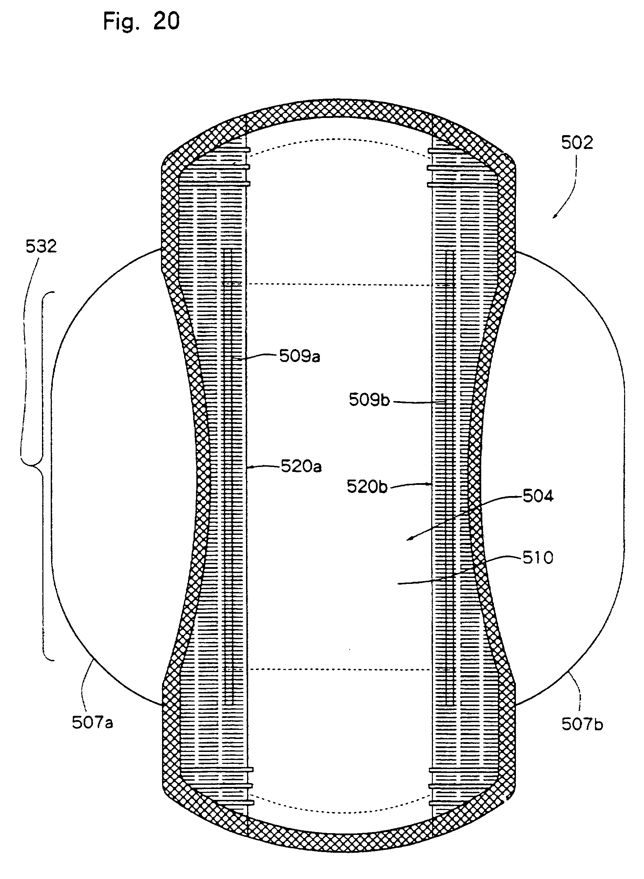

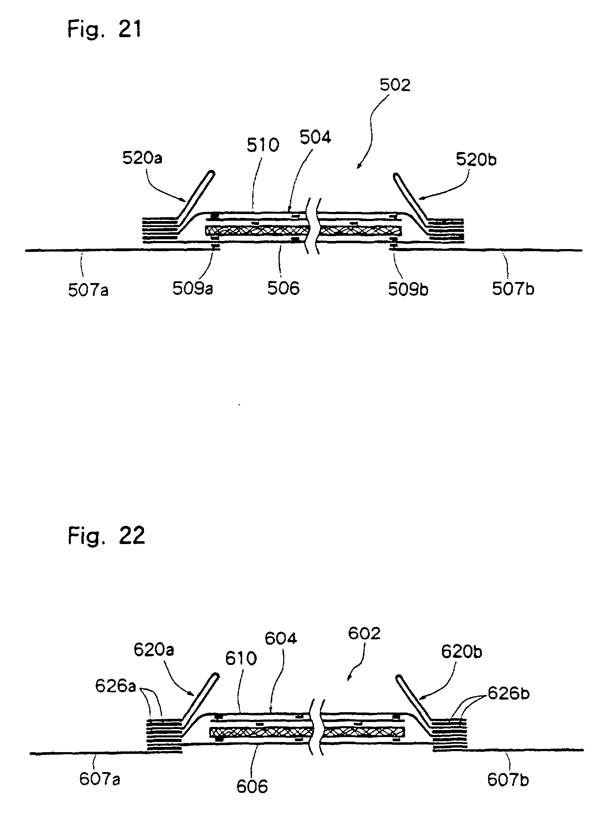

Figs. 20 and 21 show a modified example of the napkin 2 illustrated in Figs. 1 to 3. In a napkin 502 as shown in Figs. 20 and 21, a pair of wing pieces 507a and 507b are disposed under the undersurface of a backsheet 506, i.e., its outer surface. In the state illustrated in Fig. 20 in which elastic pieces 520a and 520b are elongated to make the entire napkin 502 flat, each of the pair of wing pieces 507a and 507b has a widthwise inward edge extending longitudinally straightly, and is joined to the backsheet 506 at areas 509a and 509b of thermal bonding extending along the inward edge The areas 509a and 509b of thermal bonding are delineated by solid lines in Fig. 20, and indicated by heavy solid lines in Fig. 21, for convenience's sake. Each of the pair of wing pieces 507a and 507b may be formed of a suitable material, such as the same material as for a primary topsheet layer 510 of a topsheet 504 or the backsheet 506. If desired, the wing pieces 507a and 507b may be formed of an elastic material as are the elastic pieces 520a and 520b. In this case, they may be thermally bonded to the backsheet 506 in a non-elongated state or in a longitudinally slightly elongated state. If desired, the wing pieces 507a and 507b can be joined to the backsheet 506 by ultrasonic welding or adhesive bonding, instead of thermal bonding. In a longitudinally central portion 532 of the napkin 502, the pair of wing pieces 507a and 507b are caused to extend widthwise outwardly beyond the topsheet 504 and the backsheet 506, and their widthwise outward regions are used as wings to be folded back onto the outside surface of panties. Where necessary, a pressure sensitive adhesive may be applied to the undersurface of the widthwise outward regions of the wing pieces 507a and 507b (the surface to be contacted with the outside surface of panties when these regions are folded back), and the pressure sensitive adhesive may be covered with a silicone-coated paper (not shown). The other points of constitution for the napkin 502 illustrated in Figs. 20 and 21 are substantially the same as the constitution of the napkin 2 depicted in Figs 1 to 3.

In a napkin 602 illustrated in Fig. 22, the widthwise inward edge of a pair of wing pieces 607a and 607b extend along the areas 626a and 626b of thermal bonding between a primary topsheet layer 610 of a topsheet 604 and elastic pieces 620a and 620b, and are thermally bonded to a backsheet 606 at areas consistent with the areas 626a and 626b of thermal bonding. The other constitution for the napkin 602 is substantially the same as that for the napkin 502 illustrated in Figs. 20 and 21.

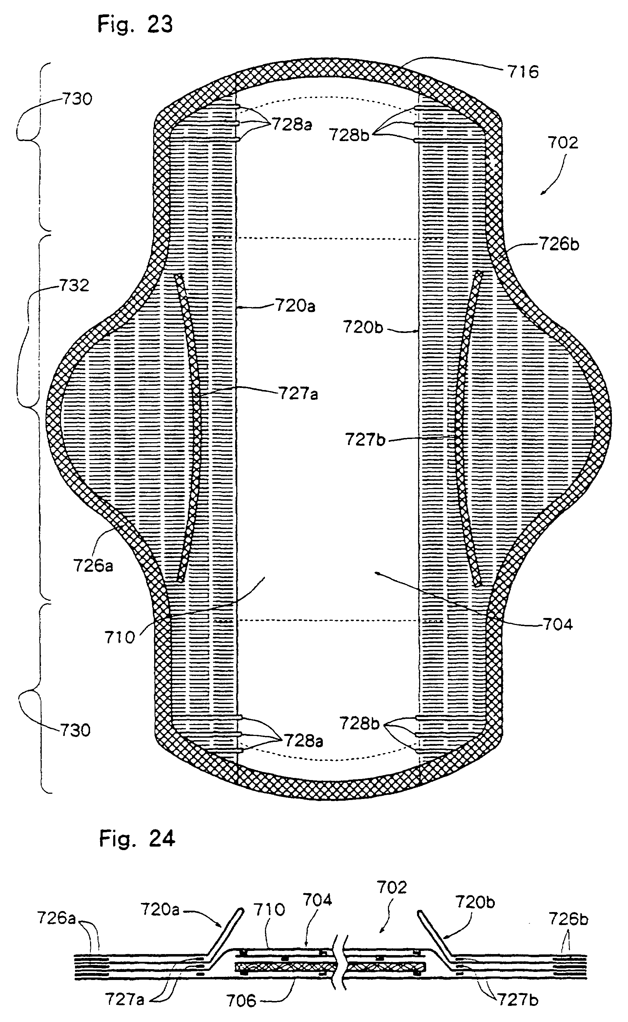

Figs. 23 and 24 show still another modified example of the napkin 2 illustrated in Figs. 1 to 3. In a napkin 702 shown in Figs. 23 and 24, a primary topsheet layer 710 of a topsheet 704, a backsheet 706, and elastic pieces 720a and 720b are caused to extend widthwise outwardly on both sides of a longitudinal central portion 732 of the napkin 702, and such extensions are in a nearly semicircular form. The primary topsheet layer 710 of the topsheet 704 and the backsheet 706 are substantially the same in shape, and are joined together at the area 716 of thermal bonding extending along their entire peripheral edges. Each of the elastic pieces 720a and 720b has the respective layers of the two-layer structure thereof thermally bonded together, and is thermally bonded to the primary topsheet layer 710 of the topsheet 704, at the areas 726a and 726b of thermal bonding extending along the widthwise outward edges and longitudinally opposite edges thereof. The areas 726a and 726b of thermal bonding are substantially conformed to the main part of the above-mentioned area 716 of thermal bonding between the primary topsheet layer 710 of the topsheet 704 and the backsheet 706 (i.e., that part in the area 716 of heating which excludes the widthwise central area of the longitudinally opposite edges where the elastic pieces 720a and 720b are not present) Furthermore, each of the elastic pieces 720a and 720b is thermally bonded to the primary topsheet layer 710 of the topsheet 704 at additional areas 727a and 727b of thermal bonding extending arcuately in the widthwise central portion of each of the elastic pieces 720a and 720b in the longitudinally central portion 732. As in the case of the napkin 2 shown in Figs. 1 to 3, each of the elastic pieces 720a and 720b has the respective layers of the two-layer structure thereof thermally bonded together, and is thermally bonded to the primary topsheet layer 710 of the topsheet 704, at three sites 728a and 728b of thermal bonding delineated in solid lines in Figs. 23, in each of the longitudinally opposite end portions 730, by operating a thermal bonding tool (not shown) at these sites.

In the napkin 702 shown in Figs. 23 and 24 as well, the elastic pieces 720a and 720b are situated on the topsheet 704 and thermally bonded, in an elongated state, to the topsheet 704 at the areas 726a and 726b of thermal bonding, the additional areas 727a and 727b of thermal bonding and the sites 728a and 728b of thermal bonding. After such thermal bonding, the force that has kept the elastic pieces 720a and 720b elongated is released. By so doing, the non-thermally bonded portions of the elastic pieces 720a and 720b elastically contract, and the elastic pieces 720a and 720b try to restore their original lengths at least partially Owing to this contracting action generated in the elastic pieces 720a and 720b, the topsheet 704, the backsheet 706 and the core 708 are displaced to extend upwardly Inclinedly toward the longitudinally opposite ends in each of the longitudinally opposite end portions 730. Thus, the napkin 702 is brought into the shape of a cup as a whole. In addition, in the longitudinally central portion 732, as illustrated in Fig. 24, those portions of the elastic pieces 720a and 720b which are widthwise inward of the additional areas 727a and 727b of thermal bonding are caused to extend upwardly inclinedly in a widthwise inward direction (accordingly, toward the aforementioned fold-back lines 722a and 722b). Thus, cuffs which function as barriers for preventing body fluids from leaking widthwise outwardly are formed on both sides of the longitudinally central portion 732 of the napkin 702. Those portions of the primary topsheet layer 710 of the topsheet 704, the backsheet 706 and the elastic pieces 720a and 720b which are present widthwise outwardly of the additional areas 727a and 727b of thermal bonding are used as wings to be folded back onto the outside surface of panties. Where necessary, a pressure sensitive adhesive may be applied to the undersurface of the widthwise outward regions of the backsheet 706 (the surface to be contacted with the outside surface of panties when these regions are folded back), and the pressure sensitive adhesive may be covered with a silicone-coated paper (not shown). If desired, in the longitudinally central portion 732 of the napkin 702, only the elastic pieces 720a and 720b and the backsheet 706 may be caused to extend widthwise outwardly (in other words, in the longitudinally central portion 732 of the napkin 702, the primary topsheet layer 710 of the topsheet 704 is not caused to extend widthwise outwardly, while both side edges of the primary topsheet layer 710 are situated inwardly of both side edges of the elastic pieces 720a and 720b as well as the backsheet 706). In this case, the wings to be folded back onto the outside surface of panties are formed from the widthwise outward regions of the elastic pieces 720a and 720b as well as the backsheet 706.

That constitution of the napkin 702 illustrated in Figs. 23 to 24 which is other than that described above may be substantially the same as the constitution of the napkin 2 shown in Figs. 1 to 3. Its explanation will be omitted herein to avoid overlapping.

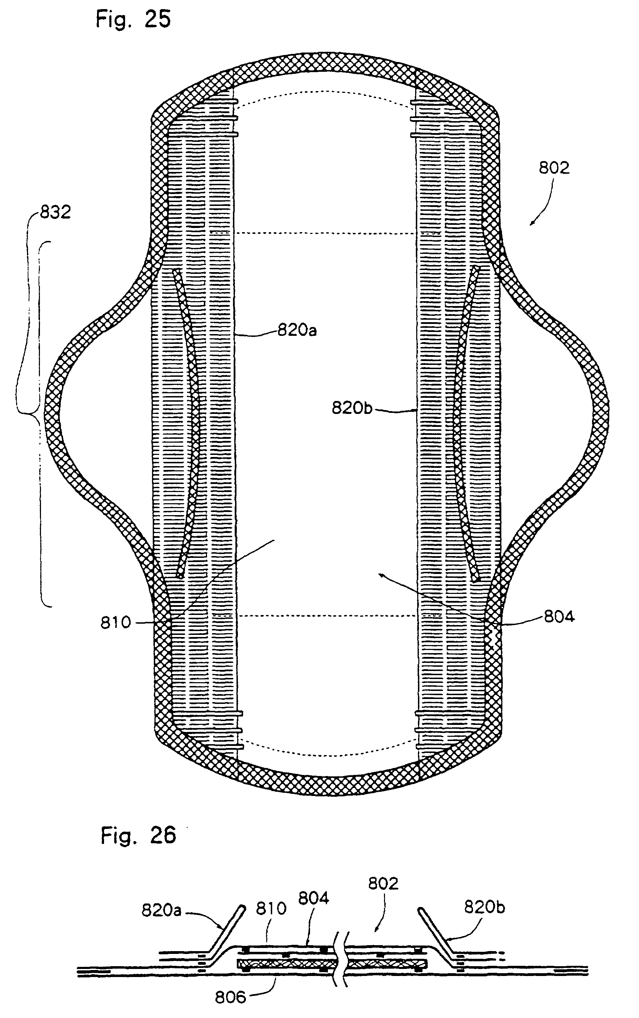

Figs. 25 and 26 show a modified example of the napkin 702 illustrated in Figs. 23 and 24. In a napkin 802 as shown in Figs. 25 and 26, in a longitudinally central portion 832 of the napkin 802, a primary topsheet layer 810 of a topsheet 804 and a backsheet 806 are caused to extend widthwise outwardly, while each of a pair of elastic pieces 820a and 820b is not caused to extend widthwise outwardly. Therefore, the portions for use as the wings to be folded back onto the outside surface of panties are formed from two layers, the primary topsheet layer 810 of the topsheet 804 and the backsheet 806. Except these points, the napkin 802 shown in Figs. 25 and 26 is substantially the same as the napkin 702 depicted in Figs. 23 and 24.

The above-described napkins 2, 102, 202, 302, 402, 502, 602, 702, 802 can be produced by, although not limited to, an arbitrary method. However, the following points should be noticed in regard to manufacturing: Generally, the absorbent core is thicker than the topsheet and the backsheet. Thus, when the core is joined to required sites of the topsheet and/or the backsheet, the napkin is relatively thick at the portions where the core is present, and relatively thin at the portions where the core is not present, thereby creating considerable differences in thickness between these two types of portions. Hence, when the elastic pieces elongated as required are joined after joining of the core to the topsheet and/or the backsheet, fully satisfactory joining tends to be hampered due to the above differences in thickness. In the present invention, therefore, a pair of second webs for forming a pair of elastic pieces are joined at predetermined sites to a first web for forming the topsheet (if the topsheet is composed of a primary topsheet layer and a secondary topsheet layer, only the primary topsheet layer, or both the primary topsheet layer and the secondary topsheet layer joined thereto) or the backsheet before the core is joined Such joining can be performed by conveying the first web, fed by a roll, by a plurality of roller pairs along a required path, and simultaneously conveying the second webs, fed by a roll, by a plurality of roller pairs along a required path, positioning all these webs as required, and joining them at predetermined sites. The second webs need to be joined in an elongated state to the first web. Thus, during conveyance of the second webs by the plurality of roller pairs, the peripheral speed of the roller pair upstream of the predetermined sites is set to be lower than the peripheral speed of the roller pair downstream of the predetermined sites. Because of this difference in the peripheral speeds of the roller pairs, the second webs are elastically elongated. Then, the second webs are positioned in a required relationship with the first web. These are measures of importance. If both the first web and the second webs are composed of a thermoplastic film, the joining of the first web and the second webs can be performed by thermal bonding while causing a thermal bonding tool to act on the first and second webs at required sites of joining. If the topsheet includes not only a primary topsheet layer but a secondary topsheet layer, it would be convenient to join the secondary topsheet layer to the primary topsheet layer beforehand, for example, via an adhesive. Then, a third web for forming the backsheet or the topsheet which third web has the core joined thereto beforehand at predetermined spaced apart locations is joined to the first web and/or the second webs joined in an elongated state to the first web. Such joining can also be carried out advantageously by conveying the third web, fed by a roll, by a plurality of roller pairs along a required path, and positioning it relative to the first and second webs as required. If the third web is also composed of a thermoplastic film, its joining to the first web and/or the second webs can be performed by thermal bonding while causing a thermal bonding tool to act at required sites of joining. If desired, instead of joining the core (and the secondary topsheet layer) to the third web beforehand, it is possible to join the core (and the secondary topsheet layer), at spaced apart locations, to the first web having the second webs joined thereto, and then join the third web to the first web and/or the second webs. Afterwards. the required shapes are cut out of the first, second and third webs, whereby the napkin can be produced. Such cutting results in the release of the tensile force exerted on the second webs. Thus, the napkin is changed from the flat form to the required form of a cup as a whole owing to the contracting action generated by the elastic pieces. Furthermore, required cuffs are formed in the longitudinally central portion of the napkin.

An absorbent article constituted in accordance with the first aspect of the present invention takes the form of a cup as a whole. forms upwardly extending cuffs on both sides of the longitudinally central portion thereof, and yet produces a fully satisfactory feeling when worn.

An absorbent article constituted in accordance with the second aspect of the present invention takes the form of a cup as a whole, forms upwardly extending cuffs on both sides of the longitudinally central portion thereof, and yet produces a fully satisfactory feeling when worn, and additionally can be manufactured for a sufficiently low cost.

## Claims

1. An absorbent article comprising a liquid pervious topsheet, a liquid impervious backsheet (6), an absorbent core (8) disposed intermediate the topsheet (4) and the backsheet (6), and a pair of band-like elastic pieces (20a, 20b) arranged on both sides of the absorbent article (2) and joined to at least one of the topsheet (4) and the backsheet (6) in a longitudinally elastically elongated state; the topsheet (4), the backsheet (6), and the core (8) being caused to extend upwardly inclinedly toward the longitudinal ends of the absorbent article (2) at each of the longitudinally opposite end portions (30) thereof, and the elastic pieces (20a, 20b) being caused to extend upwardly on both sides of the longitudinally central portion (32) of the absorbent article (2), owing to the contracting action of the elastic pieces (20a,20b); said absorbent article (2) **characterized in that**:
each of the elastic pieces (20a, 20b) is formed from a web deformed at least partially in a non-planar configuration by machining, such that each of the elastic pieces (20a, 20b) includes deformed portions (36) deformed in a non-planar configuration by machining, and undeformed portions (38) retained in a planar configuration, said web consisting in a polyolefin thermoplastic film lacking the required elasticity prior to machining.

2. The absorbent article (2) of Claim 1 wherein the undeformed portions (36) of each of the elastic pieces (20a, 20b) are caused to extend longitudinally, and the contracting action of each of the elastic pieces (20a, 20b) is generated mainly by the undeformed portions (38).

3. The absorbent article (2) of Claim 2 wherein the undeformed portions (38) undergo a substantially molecular-level, deformation and the deformed portions (36) undergo a substantially geometric deformation when the elastic pieces (20a, 20b) are subjected to an applied elongation.

## Patentansprüche

1. Absorbierender Artikel (2) mit einer flüssigkeitsdurchlässigen Decklage (4), einer flüssigkeitsundurchlässigen Außenlage (6), einem zwischen der Decklage (4) und der Außenlage (6) angeordneten absorbierenden Kern (8) und einem Paar bandartiger elastischer Stücke (20a, 20b), die auf beiden Seiten des absorbierenden Artikels (2) angeordnet sind und mit der Decklage (4) und/oder der Außenlage (6) in einem longitudinal elastisch gedehnten Zustand verbunden sind, wobei die Decklage (4), die Außenlage (6) und der Kern (8) veranlasst werden aufwärts schräg in Richtung auf die longitudinalen Enden des absorbierenden Artikels (2) in jedem von den longitudinal gegenüberliegenden Endabschnitten (30) davon zu verlaufen, und wobei die elastischen Stücke (20a, 20b) veranlasst werden aufwärts auf beiden Seiten des longitudinal zentralen Abschnittes (32) des absorbierenden Artikels (2) zu verlaufen, in Folge der Kontraktionswirkung der elastischen Stücke (20a, 20b), wobei der absorbierende Artikel (2) **dadurch gekennzeichnet ist, dass** jedes der elastischen Stücke (20a, 20b) aus einer Bahn gebildet ist, die zumindest teilweise in einer nicht-planaren Konfiguration durch maschinelle Bearbeitung deformiert ist, so dass jedes der elastischen Stücke (20a, 20b) umfasst deformierte Abschnitte (36), die in einer nicht-planaren Konfiguration durch maschinelle Bearbeitung deformiert sind, und nichtdeformierte Abschnitte (38), die in einer planaren Konfiguration gehalten werden, wobei die Bahn aus einer thermoplastischen Polyolefin-Folie besteht, der die erforderliche Elastizität vor der maschinellen Bearbeitung fehlt.

2. Absorbierender Artikel (2) nach Anspruch 1, wobei die nicht-deformierten Abschnitte (36) von jedem der elastischen Stücke (20a, 20b) veranlasst werden longitudinal zu verlaufen und die Kontraktionswirkung von jedem der elastischen Stücke (20a, 20b) hauptsächlich durch die nicht-deformierten Abschnitte (38) erzeugt wird.

3. Absorbierender Artikel (2) nach Anspruch 2, wobei die nicht-deformierten Abschnitte (38) eine im wesentlichen Molekularniveau-Deformation erfahren und die deformierten Abschnitte (36) eine im wesentlichen geometrische Deformation erfahren, wenn die elastischen Stücke (20a, 20b) einer aufgebrachten Dehnung ausgesetzt sind.

## Revendications

1. Article absorbant (2) comprenant une feuille de dessus (4) perméable aux liquides, une feuille de fond (6) imperméable aux liquides, une âme absorbante (8) disposée entre la feuille de dessus (4) et la feuille de fond (6), et une paire de pièces élastiques du type bande (20a, 20b) disposées des deux côtés de l'article absorbant (2) et réunies à au moins l'une des feuille de dessus (4) et feuille de fond (6), dans un état allongé dans la direction longitudinale ; la feuille de dessus (4), la feuille de fond (6) et l'âme étant entraînées à s'étendre d'une manière inclinée vers le haut, en direction des extrémités longitudinales de l'article absorbant (2), à chacune des parties d'extrémité (30) de celui-ci, opposées dans la direction longitudinale, et les pièces élastiques (20a, 20b) étant entraînées à s'étendre vers le haut, des deux côtés de la partie centrale (32) de l'article absorbant (2) dans la direction longitudinale, sous l'effet de l'action de contraction exercée par les pièces élastiques (20a, 20b) ; ledit article absorbant (2) étant **caractérisé en ce que** :
chacune des pièces élastiques (20a, 20b) est formée d'une nappe déformée au moins partiellement en une configuration non plane par un usinage, de telle sorte que chacune des pièces élastiques (20a, 20b) comprenne des parties déformées (36), déformées en une configuration non plane par un usinage, et des parties non déformées (38), maintenues dans une configuration plane,
ladite nappe étant constituée d'un film thermoplastique à base de polyoléfine, ne présentant pas l'élasticité requise avant l'usinage.

2. Article absorbant (2) selon la revendication 1, dans lequel les parties non déformées (36) de chacune des pièces élastiques (20a, 20b) sont amenées à s'étendre dans la direction longitudinale, et l'action de contraction de chacune des pièces élastiques (20a, 20b) est générée principalement par les parties non déformées (38).

3. Article absorbant (2) selon la revendication 2, dans lequel les parties non déformées (38) subissent une déformation essentiellement au niveau moléculaire, et les parties déformées (36) subissent une déformation essentiellement géométrique, lorsque les pièces élastiques (20a, 20b) sont soumises à un allongement appliqué.
